# EUROPEAN PATENT APPLICATION

(11) **EP 2 238 919 A2**
(43) Date of publication of application: **13.10.2010**
(21) Application number: 10250549.2
(22) Date of filing: 23.03.2010
(51) Int. Cl.: A61B 17/128, A61B 17/29, A61B 17/00

(54) **Endoscopic apparatus for manipulating tissue**

(30) Priority: 24.03.2009 US 162833 P; 11.03.2010 US 721622
(71) Applicant: Tyco Healthcare Group LP, North Haven, CT 06473 (US)
(72) Inventor: Wingardner, Thomas, North Haven, CT 06473 (US); Fowler, David, Cheshire, CT 06410 (US)
(74) Representative: Soames, Candida Jane

(57) **Abstract**

An apparatus for manipulating tissue during a surgical procedure is provided. The apparatus includes a housing including a movable handle and a release mechanism operatively coupled to a distal end of a shaft extending from the housing. The shaft defines an axial passageway configured to support at least a portion of the release mechanism. The present disclosure also provides a detachable clamp assembly operatively connectable to the distal end of the shaft and the release mechanism such that a pair of pivotally connected opposing jaw members are movable from a closed configuration for clamping tissue therebetween to an opened configuration for positioning tissue therebetween when the movable handle is moved from a first position to a second position. The clamp assembly includes a proximal end operatively coupled to a first end of a suture having a second end connected to a needle.

## Description

### CROSS-REFERENCE TO RELATED APPLICATIONS

This application claims the benefit of and priority to U.S. Provisional Patent Application No. 61/162,833, filed March 24, 2009, the entire contents of which is incorporated by reference herein.

### BACKGROUND

### 1. Technical Field

The present disclosure relates to endoscopic apparatuses and, more specifically, to an endoscopic apparatuses utilized for manipulating tissue during an endoscopic surgical procedure.

### 2. Background of the Related Art

Surgical clip or clamp systems are well known in the medical arts and are sometimes used in laparoscopic procedures for clamping tissue such that the tissue can be subsequently held, manipulated and/or stabilized.

Commercially available clamps, or clamp systems, typically include long-handled applicators that are configured for applying a clamp to internal tissue within an abdominal cavity. Typically, the clamp applicators include a pair of movable handles at the proximal end and a clamp that includes a pair of movable jaw members at a distal end. More particularly, the pair of jaw members (which are typically biased to maintain the jaw members in a normally closed position) are movable relative to one another from an open configuration for positioning tissue therebetween, to a closed configuration for clamping tissue thereafter. Such clamps typically require a clinician to insert the distal end of the applicator through a trocar sleeve (commonly referred to in the medical art as a port) secured to a patient and extended into the abdomen of a patient. Once the distal end of the applicator is properly positioned in the abdomen of a patient, the handles are actuated to open the jaw members such that the clamp may be properly secured to tissue.

In some instances, it may prove useful and/or necessary to suture the clamped tissue (or other tissue within the abdominal cavity) during a laparoscopic procedure. To this end, a clinician may utilize one or more suitable surgical instruments, such as, for example, endoscopic graspers or the like, that is configured to grasp a needle and suture operatively coupled thereto. The graspers are typically inserted through an additional port on a patient such that tissue can be subsequently sutured.

### SUMMARY

The present disclosure provides an apparatus for manipulating tissue that includes a housing including a movable handle. The housing includes a release mechanism operatively coupled to a distal end of a shaft extending from the housing. The shaft defines an axial passageway configured to support at least a portion of the release mechanism. A detachable clamp assembly operatively connects to the distal end of the shaft and the release mechanism such that a pair of pivotally connected opposing jaw members is movable from a closed configuration for clamping tissue therebetween to an opened configuration for positioning tissue therebetween when the movable handle is moved from a first position to a second position. The clamp assembly includes a proximal end operatively coupled to one end of a suture having another end connected to a needle.

In an embodiment, a detachable clamp assembly operatively connects to the distal end of the shaft and the release mechanism such that a pair of pivotally connected opposing jaw members is movable from an opened configuration for positioning tissue therebetween to a closed configuration for clamping tissue therebetween when the movable handle is moved from a first position to a second position.

The present disclosure also provides a method for performing a surgical procedure. The method includes the initial step of positioning a pair of first and second ports through an abdomen of a patient. The method includes inserting an apparatus configured for manipulating tissue through one of the pair of ports. The apparatus includes a housing including a movable handle operatively coupled to a release mechanism that operatively couples to a distal end of a shaft extending from the housing. The shaft defines an axial passageway configured to support at least a portion of the release mechanism. A detachable clamp assembly is operatively connectable to the distal end of the shaft and the actuation device such that a pair of pivotally connected opposing jaw members is movable from a closed configuration for grasping tissue therebetween to an open configuration for positioning tissue therebetween when the movable handle is moved from a first position to a second position. The clamp assembly includes a proximal end operatively coupled to one end of a suture having another end connected to a needle.

The method also includes the step of inserting a laparoscope through the second port; clamping a tissue of interest; actuating the release mechanism such that the clamping device is detached from the apparatus. Other steps of the method include removing the apparatus from the port and inserting a grasping apparatus through the one of the pair of ports that the apparatus occupied. Grasping at least a portion of the clamping device for subsequent tissue manipulation is another step of the method.

The invention may be described by reference to the following paragraphs:-

An endoscopic apparatus configured for use according to the present invention, wherein the use further includes the step of insufflating an abdominal cavity of a patient.

An endoscopic apparatus configured for use according to the present invention, wherein the step of grasping includes grasping the needle of the clamp assembly.

An endoscopic apparatus configured for use according to the present invention, wherein the use further includes the step of suturing the clamped tissue.

An apparatus for manipulating tissue during a surgical procedure comprising:
a housing including a movable handle, the housing including a release mechanism operatively coupled to a distal end of a shaft extending from the housing, the shaft defining an axial passageway configured to support at least a portion of the release mechanism; and a detachable clamp assembly operatively connectable to the distal end of the shaft and the release mechanism such that a pair of pivotally connected opposing jaw members are movable from an opened configuration for positioning tissue therebetween to a closed configuration for clamping tissue therebetween when the movable handle is moved from a first position to a second position, the clamp assembly including a proximal end operatively coupled to a first end of a suture having a second end connected to a needle.

### BRIEF DESCRIPTION OF THE DRAWINGS

Various embodiments of an endoscopic apparatus according to the present disclosure are described hereinbelow with references to the drawings, wherein:

FIGS. 1A and 1B are side, perspective views of an endoscopic instrument including a detachable clamp assembly having a pair of opposing jaw members in closed and open configurations, respectively, in accordance with an embodiment of the present disclosure;

FIGS. 1C and 1D are side, perspective views of an endoscopic instrument including a detachable clamp assembly having a pair of opposing jaw members in closed and open configurations, respectively, in accordance with another embodiment of the present disclosure;

FIG. 2 is a side, perspective view of the of the clamp assembly depicted in FIGS. 1A and 1B;

FIGS. 3A and 3B are side, perspective views of the clamp assembly depicted in FIG. 1 in use; and

FIG. 4 is a flowchart illustrating a method for performing a surgical procedure in accordance with the present disclosure.

### DETAILED DESCRIPTION

Various exemplary embodiments of the presently disclosed surgical apparatus, and method of using the same, will now be described in detail with reference to the drawings wherein like references numerals identify similar or identical elements. In the drawings and in the description which follows, the term "proximal" will refer to the end of the endoscopic apparatus that is closer to the operator during use, while the term "distal" will refer to the end of the endoscopic apparatus that is further from the operator, as is traditional and conventional in the art. In addition, the term "endoscopic apparatus" should be understood to include any surgical hand instrument formed of a biocompatible material that is suitable for the intended purpose of performing an endoscopic procedure, including but not being limited to endoscopic applicators, graspers, and the like.

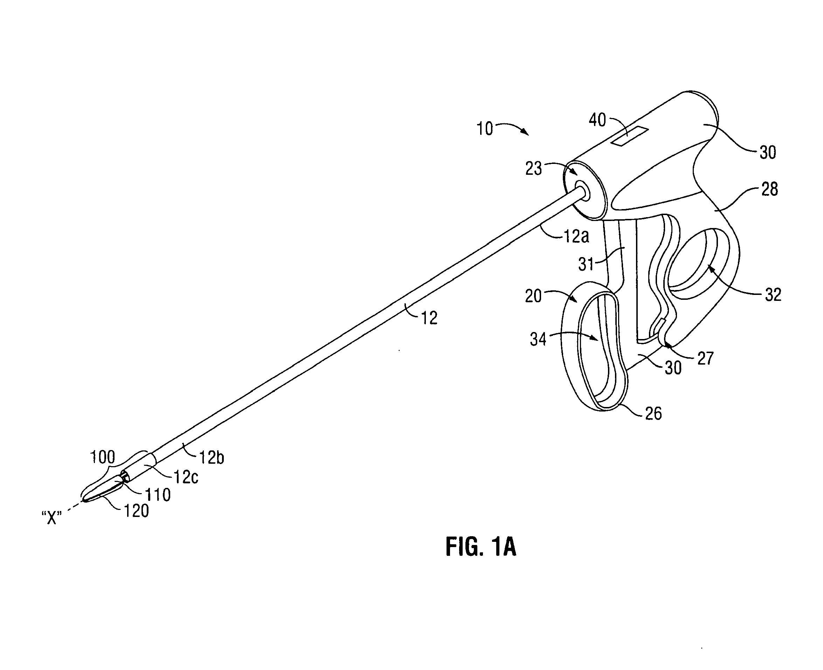

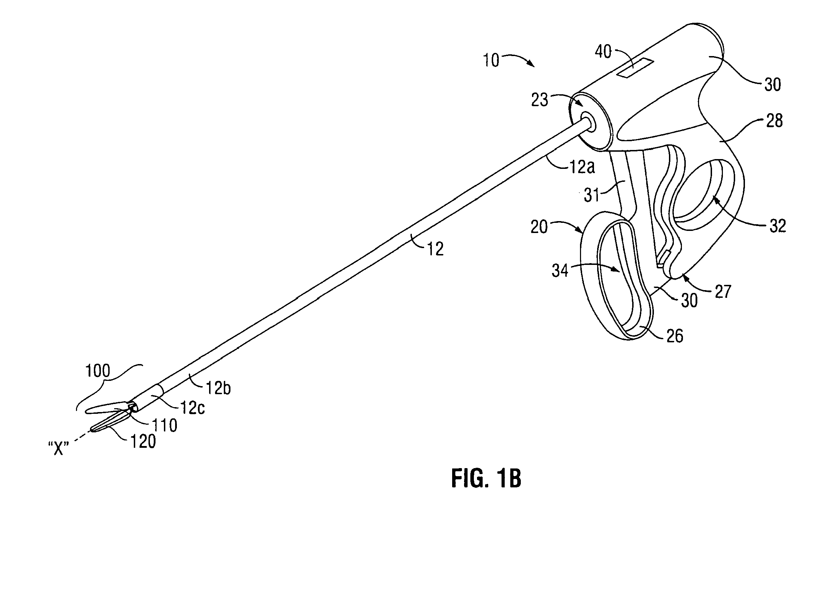

Referring to FIGS. 1A and 1B, and initially with reference to FIG. 1A, an endoscopic instrument according to an embodiment of the present disclosure is designated generally as reference numeral 10. Endoscopic instrument 10 includes an elongated shaft 12 having a proximal end 12a that mechanically couples to a housing in the form of a handle assembly 30, and a distal end 12b that is configured to receive and mechanically couple to a detachable clamp assembly 100 that includes a pair of movable, pivotably connected opposing jaw members 110 and 120.

Shaft 12 is a generally tubular hollow structure defining an axial passageway therethrough. Shaft 12 supports clamp assembly 100 at a distal end thereof such that clamp assembly 100 may be properly engaged thereto and detachable therefrom. Located at distal end 12b of shaft 12 may be one or more support structures 12c configured to releasably engage clamp assembly 100, or a portion thereof. Shaft 12, or a portion thereof, may be configured for movement relative to a longitudinal axis "X" defined therethrough. In this instance, shaft 12, or a portion thereof, may have one or more portions that are capable of articulating and/or pivoting. For a more detailed description of articulating shaft assemblies reference is made to commonly owned U.S. Patent No. 5,560,532 filed on October 8, 1993 the entirety of which being incorporated herein by reference. Elongated shaft 12 may include one or more internal electrical connections which provide electrical continuity to the clamp assembly 100 as described in more detail below.

With continued reference to FIG. 1A, handle assembly 30 mechanically engages proximal end 12a of shaft 12 and includes a movable handle 26 for imparting movement to the jaw members 110 and 120 from a clamped or closed position (FIG. 1A) wherein jaw members 110 and 120 cooperate to grasp tissue therebetween to an open position (FIG. 1B) wherein jaw members 110 and 120 are disposed in spaced relation relative to one another such that tissue may be positioned therebetween.

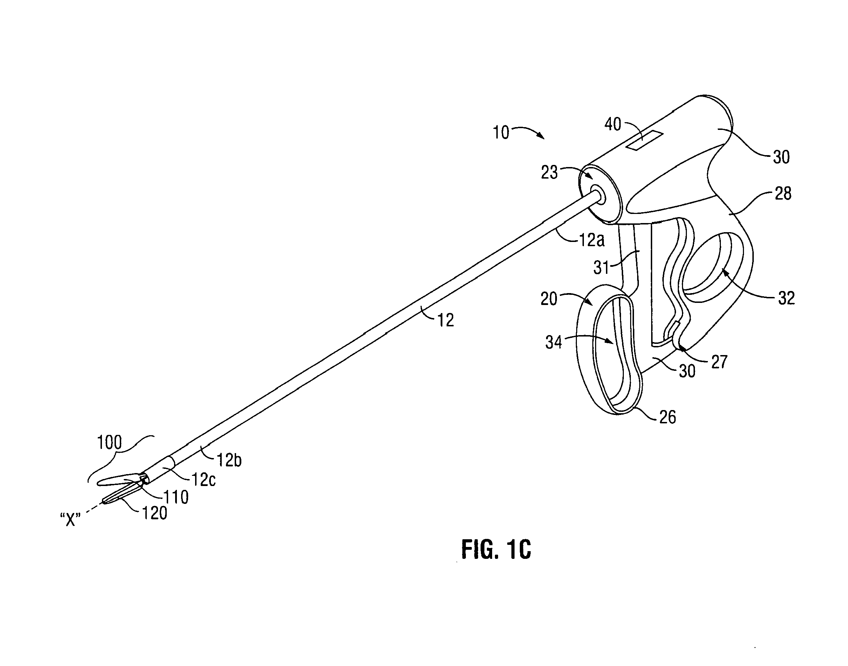

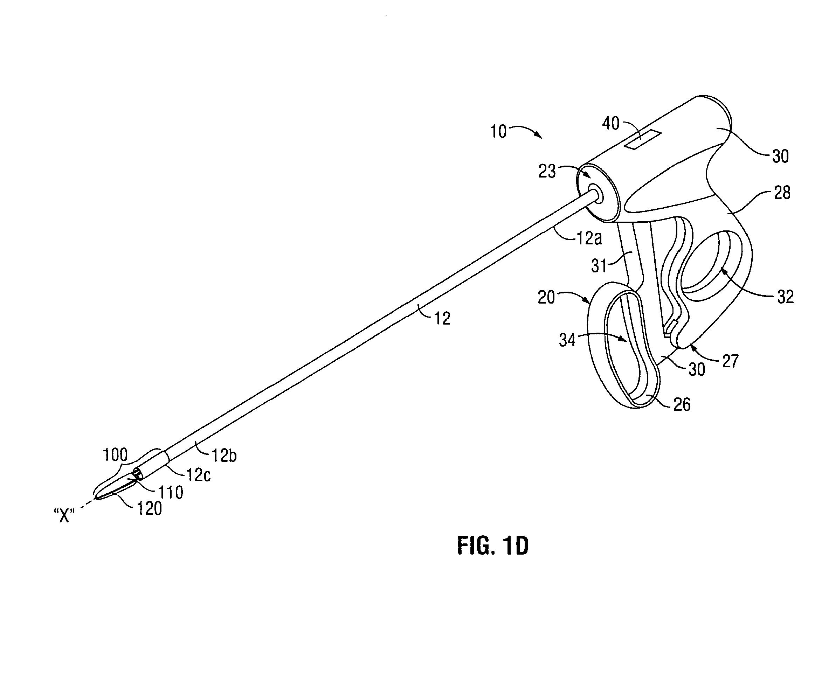

As shown in the representative drawings, movable handle 26 includes an aperture 34 configured for receiving one or more of an operator's fingers. Movable handle 26 is selectively movable from a first position (FIG. 1A) relative to a fixed handle 28 to a second position (FIG. 1B) in closer proximity to the fixed handle 28 to open jaw members 110 and 120. Similarly, moving movable handle 26 from the second position (FIG. 1B) to the first position (FIG. 1A) opens jaw members 110 and 120. In an embodiment illustrated in FIGS. 1C and 1D, movable handle 26 may be selectively movable from a first position (FIG. 1C) relative to a fixed handle 28 to a second position (FIG. 1D) in closer proximity to the fixed handle 28 to close jaw members 110 and 120, and from the second position (FIG.1D) to the first position (FIG. 1C) to open jaw members 11 and 120. In the embodiment, illustrated in FIGS. 1C and 1D, the jaw members 110 and 120 may be biased in an opened configuration. The internal mechanically cooperating components associated with the movable handle 26 to impart movement of the jaws 110, 120 of the clamp assembly 100 is commonly known and may include any number of gears, links, springs, and/or rods such that endoscopic device 30 may function as intended. In embodiments, attached to movable handle 26 is a guide 30. Guide 30 serves to maintain movable handle 26 in alignment with fixed handle 28. To this end, fixed handle includes a channel 27 that extends proximally for receiving guide 30 of movable handle 26. It is contemplated that additional mechanisms, such as, for example, hydraulic, semi-hydraulic and/or gearing systems may be employed to control and/or limit the movement of handle 26 relative handle 28.

Fixed handle 28 includes an aperture 32 configured for receiving one or more of an operator's fingers (e.g., a thumb). Fixed handle 28 provides a gripping surface for an operator's hand such that an operator may effectively manipulate the endoscopic apparatus 10 internal or external a patient.

While the drawings depict movable handle 26 and fixed handle 28 having apertures 34, 32, respectively, it is within the purview of the present disclosure that one or both of the handles 26, 28 may have solid configurations.

In addition to imparting movement to the jaws 110 and 120, handle assembly 30 may also be configured to detach clamp assembly 100 from the distal end 12b of shaft 12. A release mechanism in the form of a button 40 is in mechanical and/or electrical communication with handle assembly 30 for selectively causing clamp assembly 100 to detach from the distal end 12b of shaft 12 when button 40 is actuated (e.g., via pressing, pushing, sliding, or any other suitable actuating motions). The internal mechanically cooperating component(s) associated with each of the release mechanism 40 and/or shaft 12 to detach the clamp assembly 100 from the distal end 12b of shaft 12 is commonly known and may include any number of gears, links, drive rods, springs, and so forth such that endoscopic apparatus 10 may function as intended. Button 40 may include any number of grooves, ribs, protrusions and the like configured to facilitate actuation thereof.

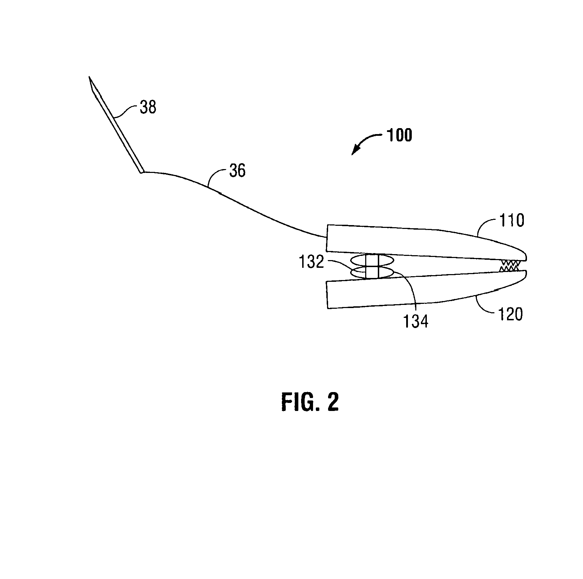

With reference now to FIG. 2, the clamp assembly 100 of the present disclosure will be described. Clamp assembly100 includes opposing jaw members 110 and 120 and suture 36 having a needle 38 operatively coupled to a proximal end thereof. As noted above, clamp assembly 100 is operatively supported at the distal end 12b of shaft 12 such that clamp assembly 100 may be detachable therefrom. With this purpose in mind, clamp assembly 100 is in operative mechanical communication with button 40 and/or movable handle 26 of handle assembly 28 and may include one or more structures that are configured to releasably engage the one or more support structures located at the distal end 12b of shaft 12. For example, clamp assembly 100 may include any number of springs, resilient members, cams and cam slots, grooves, levers, and the like (none of which being shown) such that endoscopic apparatus 10 may function as intended. In an embodiment, clamp assembly 100 is spring-loaded into the one or more support structures of shaft 12 and detachable therefrom upon actuation of button 40.

Clamp assembly 100 includes a pair of movable jaw members 110, 120 each having a contact plane for engaging tissue when jaw members 110, 120 are closed. In embodiments, the contact plane associated with each of jaw members 110, 120 includes one or more serrations or teeth configured to facilitate griping of tissue, as best seen in FIG. 2.

As noted above, jaw members 110, 120 are biased in a closed configuration and are movable and pivotably connected from a closed configuration to an open configuration by way of movable handle 26. To this end, jaw members 110, 120 are joined at a hinge or other suitable structure 132, which may include a pin or rivet (not shown). In embodiments, the hinge may be a living hinge. To facilitate biasing the jaw members 110, 120 in a closed configuration clamp assembly 100 may include one or more biasing structure (e.g., spring 134) that is in operative communication with one or both of the jaw members 110, 120. Various spring configurations that may be employed with the present disclosure include torsion, coil, leaf, compression and so forth. One skilled in the art will appreciate that other suitable structure capable of biasing the jaw members 110, 120 in a closed configuration may be utilized with the clamp assembly 100 of the present disclosure, such as, for example, rubber or other elastic structure that is capable of storing mechanical energy.

As noted above, in the embodiment illustrated in FIGS. 1C and 1D, the jaw members 110 and 120 may be biased in an opened configuration. In this embodiment, the clamp assembly 100 may include one or more locking or clamping mechanisms (not shown) that are configured to maintain the jaw members 110 and 120 in a substantially closed configuration after the jaw members 110 and 120 grasp tissue.

With continued reference to FIG. 2, a suture 36 operatively couples needle 38 to clamp assembly 100. As shown, suture 36 is coupled to a proximal end of clamp assembly 100. While the drawings depict a clamp assembly 100 that includes one suture 36 having a corresponding needle 38 coupled thereto, it is within the purview of the present disclosure that the clamp assembly 100 includes a plurality of sutures 36 having a plurality of corresponding needles 38 coupled thereto.

Suture 36 may be coupled to clamp assembly 100 by any suitable means known in the art. For example, suture 36 may be tied to one or more structures (e.g., posts, hooks, apertures and the like, which may include or be filled with an adhesive) associated with clamp assembly 100. Suture 36 may be composed of any suitable biocompatible material known in the art and may be of the absorbable or non-absorbable type. Commonly used absorbable sutures suitable for use with the present disclosure may include but are not limited to those composed of surgical gut, polyglycolic acid, and chromic suture material. Commonly used non-absorbable sutures suitable for use with the present disclosure may include but are not limited to those composed of monofilament nylon, polymer polypropylene, and so forth. Suture 36 may have a degree of elasticity, or suture 36 may be substantially rigid. In embodiments, suture 36 may be braided.

Needle 38 may be any type of surgical needle known in the art that is capable of coupling to suture 36 for manipulation thereof. For example, needle 38 may be of the "Traumatic" or "Atraumatic" types of needles. In the instance where needle 38 is "Atraumatic", needle 38 may be permanently swaged to the suture 36, in which instance needle 38 would be detachable from suture 36 by way of cutting, or needle 38 may be controlled-released swaged to the suture 36 (commonly referred to in the art as "pop-off" swaged), in which instance needle 38 would be detachable from the suture 36 by way of a brisk or fast tug. Needle 38 may have a straight configuration; half-curved or ski configuration; ¼,⅜,½,*or* ⅝ circle configuration; compound curve configuration; or any other suitable configuration. Needle 38 may have any suitable point geometry known in the art including but not limited to trocar points, blunt points, and spatula points. Needle 38 may be configured for forward or reverse cutting.

When clamp assembly 100 is attached to the distal end 12b of shaft 12, needle 38 may be supported within shaft 12 by any suitable structure (not shown). To this end, and as noted above, shaft 12 may include one or more support structures configured to releasably engage needle 38 and/or suture 36 such that needle 38 and/or suture 36 are each prevented or impeded from moving prior to detachment of clamp assembly 100 from shaft 12. For example, located at the distal end 12b of shaft 12 may be internal support structure (not shown) configured to receive a portion of needle 38 via a press or friction fit. In this instance, the pulling force caused by clamp assembly 100 detaching from shaft 12 causes the needle 38 and/or suture 36 to detach from the support structure for manipulation thereafter.

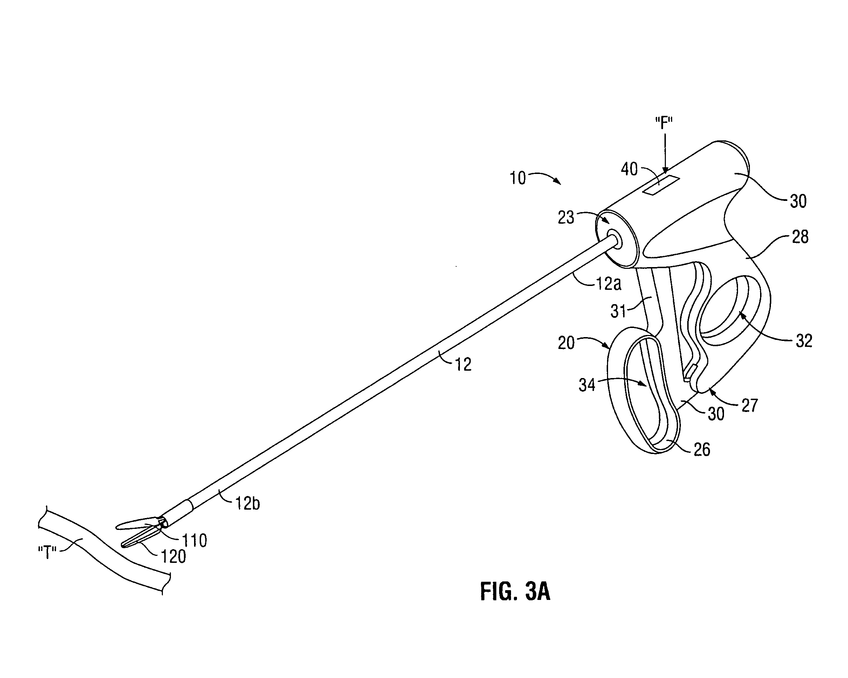

In use, clamp assembly 100 is initially in a closed configuration (FIG. 1A). Prior to grasping tissue an operator moves movable handle 26 proximally toward fixed handle 28 which, in turn, causes jaw members 110, 120 to move away from each other such that the jaw members 110, 120, are in an open configuration (FIGS. 1B and 3A).

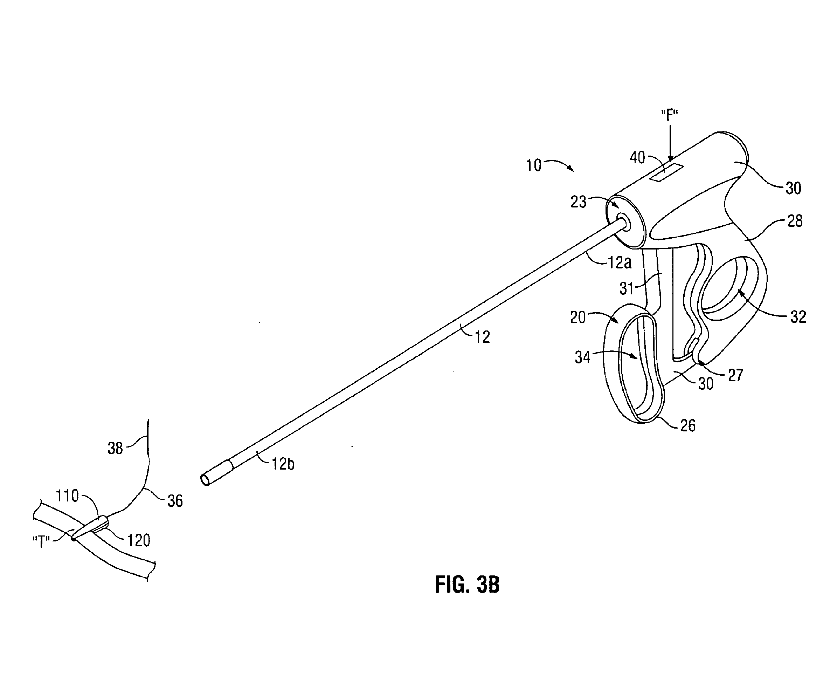

Once tissue is properly positioned between the jaw members 110, 120, movable handle 26 is released, which, in turn causes the jaw members 110, 120 to return to their initial closed configuration such that the clamp assembly 100 is now attached or clamped to the tissue (FIG. 3B).

Alternatively, the jaw members 110 and 120 may be configured in a manner according to the embodiment illustrated in FIGS. 1C and ID. In this instance, after tissue is properly positioned between the jaw members 110 and 120, movable handle 26 may be moved proximally toward fixed handle 28, which, in turn causes the jaw members 110 and 120 to move from their initially opened configuration toward each other such that the jaw members 110 and 120 are in a closed configuration (FIG.1C) and clamped to tissue. As noted above, a locking mechanism may be employed to maintain the jaw members 110 and 120 in the closed configuration and attached to tissue.

After the tissue is properly clamped, an operator may actuate button 40 (e.g., presses in the direction indicated by force vector "F", as best seen in FIG. 3B) which, in turn, causes the clamp assembly 100 including the suture 36 and needle 38 attached thereto to detach from the distal end 12b of shaft 12. If desired, another clamp assembly may be attached and/or loaded onto endoscopic apparatus 10 such that another tissue of interest may be clamped.

When clamp assembly 100 is detached, an operator may subsequently grasp the needle 38 (by way of endoscopic apparatus 10 fitted with another clamp assembly 100, or other suitable surgical apparatus, such as, for example, a grasper (not shown) such that tissue can be manipulated (e.g., sutured).

From the foregoing and with reference to the various figure drawings, those skilled in the art will appreciate that certain modifications can also be made to the present disclosure without departing from the scope of the same. For example, endoscopic apparatus 10 may be adapted to connect to a source of electrosurgical energy such that an electrosurgical procedure may be performed. In this instance, the endoscopic apparatus 10 may be provided with an electrical connector for connection to an electrosurgical generator. The generator may supply thermal, ultrasonic and/or RF energy to the jaw members 110, 120 of the clamp assembly 100 through a connector operatively coupled to the clamp assembly 100. In embodiments, the suture 36 and needle 38 may be configured to provide a path for the flow of electrosurgical energy to the jaw members 110, 120.

It is contemplated, that handle 30 may include a rotating assembly for controlling the rotational movement of clamp assembly 100 about a longitudinal axis "X" defined along shaft portion 12. In use, as the rotating assembly is rotated about the longitudinal "X" axis, clamp assembly 100 is correspondingly rotated about the longitudinal "X" axis. One such type of rotating assembly is disclosed in commonly-owned U.S. Patent No. 7,101,372 entitled "VESSEL SEALER AND DIVIDER" and U.S. Patent No. 7,156,846 entitled "VESSEL SEALER AND DIVIDER FOR USE WITH SMALL TROCARS AND CANNULAS" the contents of each being incorporated by reference herein in their entirety.

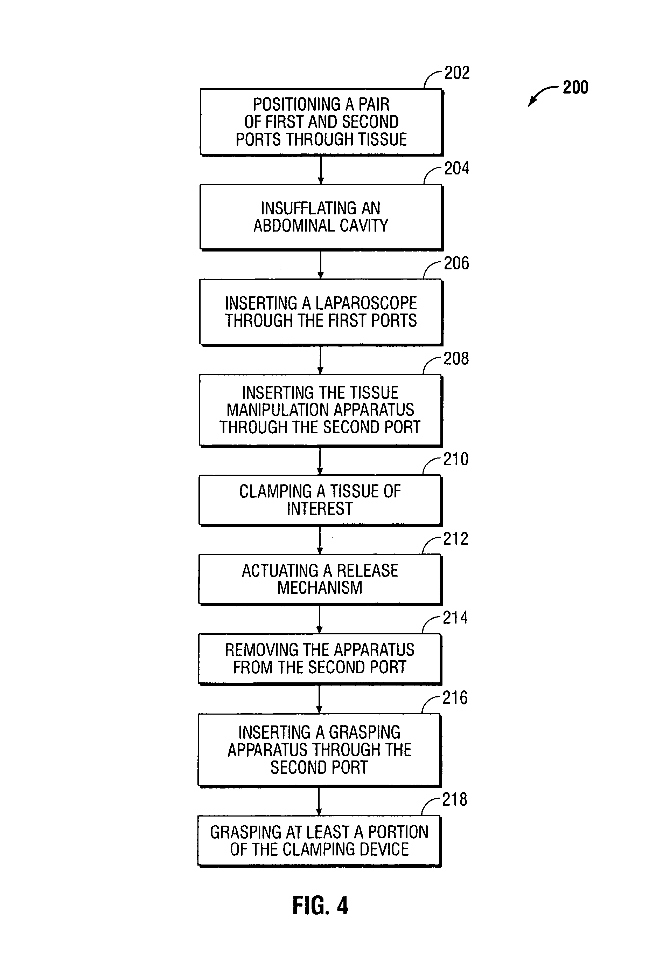

With reference now to FIG. 4 a method 200 for performing an endoscopic procedure (e.g., laparoscopic procedure) is illustrated. At step 202, a pair of first and second trocar sleeves or ports of well-known construction are inserted through the abdominal wall using known techniques. The ports provide a sealed entryway into the abdominal cavity through which surgical instruments may be inserted. For laparoscopic procedures the abdominal cavity will be distended using insufflation or other technique (step 204) and a laparoscope will be inserted through the first port and positioned within the body cavity (step 206) to facilitate visualization of the surgical site. At step 208, an endoscopic apparatus 10 for manipulating tissue is inserted through the second port.

As described in detail hereinabove, movable handle 26 may be moved proximally toward fixed handle 28 such that the jaw members 110, 120 move from their closed configuration to their open configuration. The open jaw members 110, 120 are then positioned over a tissue location "T" by longitudinal, rotational and/or angular movement of endoscopic apparatus 10.

When clamp assembly 100 is positioned over a tissue location 126, movable handle 26 is released so as to close jaw members 110, 120 on the tissue such that tissue is now clamped therebetween (step 210).

When tissue is clamped, button 40 is depressed in the direction indicated by force arrow "F" which, in turn, causes at least a portion clamp assembly 100 to detach from the distal end 12b of shaft 12 (step 212).

At step 214, once clamp assembly 100 is detached from the endoscopic apparatus 10, endoscopic apparatus 10 is removed from the abdominal cavity.

A suitable endoscopic apparatus (e.g., endoscopic grasper, not shown) is inserted through the second port (step 216) and into the abdominal cavity such that needle 38 of clamp assembly 100 may be grasped (step 218) for tissue manipulation thereafter.

If needed, an additional clamp assembly 100 may subsequently be attached to a free end of the distal end 12b of shaft 12 and the above described steps may be repeated

While the above is a complete description of the preferred embodiments of the invention, various alternatives, modifications and equivalents may be used. Therefore, the above description should not be taken as limiting the scope of the invention which is defined by the appended claims.

## Claims

1. An apparatus for manipulating tissue during a surgical procedure comprising:
a housing including a movable handle, the housing including a release mechanism operatively coupled to a distal end of a shaft extending from the housing, the shaft defining an axial passageway configured to support at least a portion of the release mechanism; and
a detachable clamp assembly operatively connectable to the distal end of the shaft and the release mechanism such that a pair of pivotally connected opposing jaw members are movable from a closed configuration for clamping tissue therebetween to an opened configuration for positioning tissue therebetween when the movable handle is moved from a first position to a second position, the clamp assembly including a proximal end operatively coupled to a first end of a suture having a second end connected to a needle.

2. An apparatus according to claim 1, wherein each of the suture and the needle are operatively housed within the axial passageway of the shaft.

3. An apparatus according to claim 1 or claim 2, wherein the clamp assembly is spring-loadable.

4. An apparatus according to claim 3, wherein the release mechanism is configured to releasably engage at least a portion of the clamp assembly.

5. An apparatus according to any preceding claim, wherein the shaft includes a holding structure configured to support at least a portion of the clamp assembly.

6. An apparatus according to claim 5, wherein the needle of the clamp assembly is releasably connected to the holding structure of the shaft.

7. A detachable clamp assembly adapted to connect to an endoscopic apparatus configured for detachment thereof comprising:
a pair of pivotally connected opposing jaw members movable from an open configuration for positioning tissue therebetween to a closed configuration for grasping tissue therebetween when the movable handle is moved from a first position to a second position, the clamp assembly including a proximal end operatively coupled to a first end of a suture having a second end connected to a needle.

8. A detachable clamp assembly according to claim 7, wherein each of the suture and the needle are operatively housed within an axial passageway of a shaft of the tissue manipulation device.

9. A detachable clamp assembly according to claim 7 or claim 8, wherein the clamp assembly is spring-loadable.

10. An endoscopic apparatus configured for use in manipulating tissue, in a method of performing a surgical procedure which apparatus comprises:
a housing including a movable handle operatively coupled to a release mechanism operatively coupled to a distal end of a shaft, the shaft defining an axial passageway configured to support at least a portion of the release mechanism; and
a detachable clamp assembly operatively connectable to the distal end of the shaft and an actuation device such that a pair of pivotally connected opposing jaw members are movable from a closed configuration for grasping tissue therebetween to an open configuration for positioning tissue therebetween when the movable handle is moved from a first position to a second position, the clamp assembly including a proximal end operatively coupled to a first end of a suture having a second end connected to a needle; and
wherein the method comprises the steps of: positioning a pair of first and second ports through an abdominal cavity of a patient; inserting the endoscopic apparatus through the first port; inserting a laparoscope through the second port;
clamping a tissue of interest
actuating the release mechanism such that the clamping device is detached from the endoscopic apparatus;
removing the endoscopic apparatus from the first port;
inserting an endoscopic grasping apparatus through the first port; and
grasping at least a portion of the clamping device for subsequent tissue manipulation.

11. An endoscopic apparatus configured for use according to claim 10, wherein the use further includes the step of insufflating an abdominal cavity of a patient.

12. An endoscopic apparatus configured for use according to claim 10 or claim 11, wherein the step of grasping includes grasping the needle of the clamp assembly.

13. An endoscopic apparatus configured for use according to claim 10 or claim 11, wherein the use further includes the step of suturing the clamped tissue.

14. An apparatus for manipulating tissue during a surgical procedure comprising:
a housing including a movable handle, the housing including a release mechanism operatively coupled to a distal end of a shaft extending from the housing, the shaft defining an axial passageway configured to support at least a portion of the release mechanism; and
a detachable clamp assembly operatively connectable to the distal end of the shaft and the release mechanism such that a pair of pivotally connected opposing jaw members are movable from an opened configuration for positioning tissue therebetween to a closed configuration for clamping tissue therebetween when the movable handle is moved from a first position to a second position, the clamp assembly including a proximal end operatively coupled to a first end of a suture having a second end connected to a needle.
